# FASCICULE DE BREVET EUROPEEN

(11) **EP 0 619 113 B1**
(45) Date de publication et mention de la délivrance du brevet: **18.06.1997**
(21) Numéro de dépôt: 94400484.5
(22) Date de dépôt: 08.03.1994
(51) Int. Cl.: A61K 7/135

(54) **Composition granulée de peroxydés utilisable pour la décoloration des cheveux et procédé pour la préparer**
Granulierte Peroxyd-Zusammensetzung zum Bleichen von Haar und Herstellungsverfahren
Granulated peroxyde composition useful for bleaching hair and process for preparation

(30) Priorité: 05.04.1993 FR 9303994
(43) Date de publication de la demande: 12.10.1994
(73) Titulaire: L'OREAL, 75008 Paris (FR)
(72) Inventeur: Millequant, Jean-Marie, F-94100 Saint-Maur (FR); Tricaud, Caroline, F-95240 Cormeilles en Parisis (FR); Gaboriaud, Anne, F-93340 Le Raincy (FR)
(74) Mandataire: Peuscet, Jacques

(56) Documents cités:
- EP-A- 0 256 127
- WO-A-92/03120
- GB-A- 2 133 983
- US-A- 3 089 824

## Description

La présente invention concerne une composition granulée à base de dérivés peroxydés, utilisable pour la décoloration des cheveux, et un procédé de préparation de ladite composition.

Il est connu de décolorer les cheveux à l'aide d'une pâte obtenue en mélangeant, au moment de l'application sur les cheveux, une composition décolorante à base de dérivés peroxydés avec de l'eau ou, mieux encore, avec de l'eau oxygénée. La composition décolorante est, de façon connue, constituée d'un dérivé peroxydé, généralement un persulfate ou un perborate de sodium, de potassium ou d'ammonium et parfois un sel d'acide percarboxylique ou un peroxyde, par exemple de baryum ou de strontium. Ces compositions contiennent également, de façon connue, des agents fortement alcalins tels que des métasilicates, des phosphates ou des carbonates alcalins ou alcalino-terreux ; elles peuvent éventuellement contenir d'autres additifs : des agents de contrôle du dégagement d'oxygène lors du mélange avec l'eau oxygénée, tels que le carbonate de magnésium ou la magnésie ; des agents tensioactifs, tels que les sulfates d'alcools gras, les alkylsulfates et les alkylbenzène sulfonates ; des agents épaississants, tels que les dérivés de cellulose, par exemple la carboxyméthylcellulose, l'amidon et ses dérivés, les gommes de guar, de xanthane et les alginates ; des colorants bleus ou violets et des parfums. De telles compositions décolorantes sont décrites, par exemple, dans "The science of hair care" par C. Zviak, Marcel Dekker Inc. 1986, pages 225 et 226.

La composition décolorante est souvent utilisée sous forme de poudre de faible granulométrie, ce qui permet une dissolution facile et rapide dans l'eau oxygénée. Mais ces compositions pulvérulentes présentent plusieurs inconvénients. En premier lieu, les compositions pulvérulentes sont constituées de poudres ayant des densités apparentes différentes et, au cours de leur manipulation et de leur stockage, il se forme une ségrégation des constituants, les plus lourds se rassemblant à la partie inférieure de l'emballage dans lequel la composition est contenue et les plus légers à la partie supérieure ; par conséquent, lors du prélèvement de la composition pour la mélanger avec l'eau oxygénée, les volumes prélevés à la partie supérieure de l'emballage et ceux prélevés à la partie inférieure ont des compositions différentes et donc un pouvoir de décoloration différent. En second lieu, les compositions sous forme pulvérulente émettent, lors de leur manipulation, des poussières qui contiennent des dérivés peroxydés et sont, par conséquent, fortement irritantes pour les poumons.

Pour résoudre ce problème, on a déjà proposé de mettre la composition décolorante sous forme granulée.

Selon WO-A 92/03120, on a proposé de granuler un persulfate et de le mélanger avec des particules, éventuellement granulées, des différents autres constituants de la composition. La granulation du persulfate peut se faire soit par pulvérisation et séchage d'une solution aqueuse de persulfate contenant éventuellement des agents tensioactifs ou des agents épaississants solubles dans l'eau, soit par pulvérisation d'une solution d'agent tensioactif ou d'agent épaississant sur un lit mobile de persulfate solide. Ce procédé, s'il peut permettre, a priori, d'éviter la formation de poussières irritantes de persulfate ne permet certainement pas de résoudre le problème de la ségrégation des particules des différents constituants de la composition décolorante.

Dans FR-A 2 044 324, on a proposé de granuler l'ensemble des constituants de la composition décolorante pulvérulente à l'aide d'un liant : la polyvinylpyrrolidone ou le glucose en solution dans un milieu aqueux, hydroalcoolique ou alcoolique. On résoud ainsi le problème de la ségrégation des différents constituants de la composition décolorante puisque les différents constituants sont présents dans un même granulé. Mais on a constaté que l'emploi de polyvinylpyrrolidone seule, comme liant, donne des granules durs mais cependant friables qui, par frottement répété, forment de fines poussières qui sont susceptibles de voler dans l'atmosphère, en particulier au moment de l'emploi. De plus, on obtient des granules ayant une granulométrie élevée comprise entre 1 et 6 mm, ce qui augmente le temps de dissolution et donne, par mélange avec l'eau oxygénée, une pâte qui reste longtemps granuleuse et dont l'application est désagréable ; de plus, cette pâte risque de donner par application sur les cheveux une décoloration non homogène et non reproductible. Pour améliorer l'utilisation des granules obtenus selon FR-A 2 044 324 on a essayé de les broyer mais il se forme au cours du broyage des poussières irritantes. De plus, le broyage augmente le coût des granules.

Selon la présente invention, on a trouvé qu'en utilisant un liant particulier, à savoir un polypropylèneglycol de poids moléculaire déterminé, éventuellement mélangé avec au moins un polyalkylèneglycol ayant un groupe alkylène en C₂ ou C₄ de poids moléculaire déterminé, on obtient des granules qui ne forment pratiquement pas de poussière par frottement. De plus, la granulation permet d'obtenir, avec un rendement élevé, des granules ayant une granulométrie comprise entre 65 µm et 800 µm, ces granules se dissolvant facilement dans l'eau oxygénée pour donner une pâte homogène et onctueuse facile à appliquer sur une chevelure. En outre, il faut noter qu'il est possible de broyer les granules ayant une granulométrie supérieure à 800 µm, sans qu'il se forme de poussière irritante.

La présente invention a, par conséquent, pour objet une composition granulée à base de dérivés peroxydés, utilisable pour la décoloration des cheveux, dans laquelle les granules ont été obtenus par granulation d'un mélange de ses différents constituants pulvérulents au moyen d'un liant, caractérisée par le fait que le liant comprend du polypropylèneglycol ayant un poids moléculaire compris entre 200 et 10 000, de préférence compris entre 2 000 et 8 000, les granules ayant une granulométrie comprise entre 65 µm et 800 µm.

Selon la présente invention, le polypropylèneglycol peut être utilisé comme liant, seul ou en mélange avec une faible quantité d'au moins un polyalkylèneglycol ayant un groupe alkylène en C₂ ou C₄ (dit ci-après "polyalkylèneglycol en C₂ ou C₄") ayant un poids moléculaire compris entre 200 et 30 000 ; le polyalkylèneglycol peut donc être du polyéthylèneglycol et/ou du polybutylèneglycol. De préférence, le polyéthylèneglycol a un poids moléculaire compris entre 200 et 4 000, et le polybutylèneglycol a un poids moléculaire compris entre 200 et 30 000.

Selon l'invention, de préférence, la concentration globale en liant (polypropylèneglycol seul ou mélange de polypropylèneglycol et de polyalkylèneglycol(s) en C₂ ou C₄) dans la composition granulée ne dépasse pas 25 % en poids par rapport au poids total de la composition. La concentration en polypropylèneglycol est avantageusement comprise entre 10 et 20 % en poids par rapport au poids total de la composition. La concentration en polyalkylèneglycol(s) en C₂ ou C₄, en mélange avec le polypropylèneglycol, ne dépasse pas, de préférence, 5 % en poids par rapport au poids total de la composition.

Selon l'invention, la composition granulée doit avoir une granulométrie comprise entre 65 µm et 800 µm. En effet, lorsque la granulométrie est supérieure à 800 µm, les granules commencent à se dissoudre difficilement dans l'eau oxygénée et leur dissolution peut ne pas être totale ; la pâte obtenue est granuleuse et n'est pas homogène. Lorsque la composition a une granulométrie inférieure à 65 µm, il se forme des poussières irritantes lors de la manipulation de la composition décolorante sous forme granulée.

Selon la présente invention, on a également trouvé que se dissolvent plus facilement dans l'eau oxygénée, les compositions granulées contenant au moins un agent lubrifiant de la famille des dérivés de cellulose, de préférence la carboxyméthylcellulose de sodium ou l'hydroxypropylméthylcellulose et/ou au moins un agent lubrifiant de la famille des stéarates alcalins ou alcalino-terreux ou de polyol. Les stéarates de polyol sont, de préférence, choisis parmi le stéarate de glycol et le stéarate de glycérol. On utilise, de préférence, un mélange de stéarate de potassium et de stéarate de glycérol. En effet, en présence d'agent(s) lubrifiant(s), les granules sont moins durs et moins friables. La quantité de dérivé(s) de cellulose présente comme lubrifiant est, avantageusement, comprise entre 1 et 10 % en poids par rapport au poids total de la composition, de préférence, 2 à 6 % ; la quantité de stéarate(s) présente est, avantageusement, comprise entre 0,1 et 5 % en poids par rapport au poids total de la composition, de préférence, 0,2 et 1 % ; la quantité globale d'agent lubrifiant est avantageusement comprise entre 1 et 10 % en poids par rapport au poids total de la composition.

La présente invention a également pour objet un procédé de préparation de la composition granulée ci-dessus définie, caractérisé par le fait que l'on prépare un lit mobile des différents constituants pulvérulents du mélange à granuler, on pulvérise sur le lit mobile du polypropylèneglycol, éventuellement mélangé avec au moins un polyalkylèneglycol en C₂ ou C₄ sous forme de solution dans un solvant et on élimine le solvant.

Ce procédé permet d'obtenir des granules de granulométrie plus homogène et, en particulier, d'éviter la formation de fines ayant une granulométrie inférieure à 65 µm. La quantité de granules ayant une granulométrie supérieure à 800 µm peut être faible mais, de toute façon, s'ils existent en quantité significative, ces granules peuvent être rebroyés sans que, pratiquement, ne se produisent des poussières irritantes. Pour régler la granulométrie à la valeur désirée, on agit sur la durée de la granulation. Celle-ci est, de façon convenable, comprise entre 3 et 15 minutes.

Le solvant peut être tout solvant compatible avec le liant, qui y est dissous. On évite cependant d'utiliser un milieu aqueux (de l'eau ou un milieu hydroalcoolique, par exemple) car la présence d'eau peut amorcer la décomposition des dérivés peroxydés et, lorsque la composition décolorante contient un dérivé d'ammonium (par exemple du persulfate d'ammonium), provoquer un dégagement d'ammoniac. Le solvant est, de préférence, un alcanol en C₁-C₄, les chlorures de méthylène ou d'éthylène, ou les mélanges de ces composés.

Le lit mobile de mélange pulvérulent sur lequel est pulvérisée la solution de liant peut être tout lit mobile connu pour la granulation : il peut être un lit obtenu dans une cuve à l'aide d'agitateur(s), notamment de pales ou de batteurs, un lit obtenu dans un tambour rotatif, ou un lit obtenu à l'aide d'un courant gazeux ascendant, tel qu'un lit fluidisé.

Le mélange pulvérulent est, de préférence, homogénéisé avant la pulvérisation de la solution de liant. Cette homogénéisation peut être effectuée dans un mélangeur annexe ou dans le lit mobile avant pulvérisation. Par exemple, dans le cas où on effectue la granulation dans une cuve, on introduit dans la cuve tous les constituants pulvérulents du mélange, on homogénéise le mélange par agitation dans la cuve avant de pulvériser la solution de liant, et on poursuit l'agitation, après en avoir éventuellement modifié les conditions, pour maintenir le mélange à granuler sous forme de lit mobile pendant la pulvérisation et la granulation. Lorsqu'on granule en lit fluidisé, le mélange pulvérulent à granuler peut être homogénéisé par agitation dans une cuve annexe, avant introduction dudit mélange dans le granulateur à lit fluidisé.

La granulation peut être effectuée en discontinu ou en continu.

Après granulation, on sépare éventuellement, d'une part, les fines ayant des dimensions inférieures à 65 µm, ces fines pouvant être recyclées, et d'autre part, les particules ayant des dimensions supérieures à 800 µm, qui sont broyées sans provoquer la formation de poussières irritantes ; le produit obtenu après broyage et classification est introduit selon sa granulométrie, soit dans le mélange pulvérulent, soit dans le produit fini.

Les exemples donnés ci-après, à titre illustratif et nullement limitatif, permettront de mieux comprendre l'invention.

### EXEMPLES 1 et 2

### 1) Préparation des granulés

On utilise un granulateur "ROTO 50 P" commercialisé par la société italienne "ZANCHETTA & C" comportant une cuve de 50 litres, muni d'un couvercle équipé de couteaux rotatifs et d'une buse de pulvérisation, muni d'un agitateur tripale en fond de cuve, d'une double enveloppe, d'une pompe à vide et d'un système de basculement de la cuve à ± 90°.

On introduit dans la cuve 13,5 kg du mélange pulvérulent à granuler. La composition du mélange à granuler est donnée dans les onze premières lignes du tableau I ci-après. On homogénéise le mélange pendant 5 minutes à l'aide du tripale tournant à 200 tours/minute et des couteaux tournant à 1 000 tours/minute. On arrête les couteaux et on poursuit l'agitation à l'aide du tripale après avoir amené sa vitesse de rotation à 160 tours/minute.

Pendant ce temps, on prépare une solution de polypropylèneglycol (PPG) de poids moléculaire 2 000 ou 6 000 respectivement pour les exemples 1 et 2, dans le dichlorométhane ; cette solution est constituée en mélangeant 1,5 kg de PPG pour 1 litre de solvant.

Dès que l'on a arrêté les couteaux comme indiqué ci-dessus, on pulvérise la solution de PPG sur le lit de particules contenu dans le granulateur, de façon à ajouter 1,5 kg de PPG au mélange pulvérulent.

Lorsque la pulvérisation est terminée, on amène la vitesse de rotation du tripale à 200 tours/minute et on fait tourner les couteaux à 1 200 tours/minute jusqu'à obtention d'un pic de puissance des moteurs de l'agitation, ce qui correspond à la fin de la granulation.

On met en route la pompe à vide, on bascule de 90° le granulateur (l'axe de la cuve est alors presque horizontal) et on chauffe la double enveloppe de façon à obtenir une température ne dépassant pas 40°C dans le lit de granules et à éliminer le solvant.

### 2) Essais sur les granules obtenus :

Quand l'élimintation du solvant est terminée, on effectue les tests suivants :
a) Mesure de la granulométrie dans l'appareil de calibration commercialisé sous la dénomination "FC" par la société italienne "ZANCHETTA & C".
b) Test de dissolution des grains :
   On prélève 10 g de poudre décolorante granulée que l'on met dans un bol non métallique. On empâte à l'aide d'une spatule avec 10 g d'eau oxygénée à 30 volumes. On détermine le temps de dissolution totale par observation à l'oeil nu du cataplasme obtenu, le temps maximum étant de 30 minutes.
c) Mesure de la "volatilité" :
   On pèse dans des pots en verre 10 g de poudre granulée des différents échantillons de poudre granulée.

On agite de la même façon (trois allers et retours de bas en haut et vice versa) et on évalue visuellement le nuage de poussière qui subsiste en prenant en compte la densité du nuage, sa persistance et la taille des particules en suspension. On définit une échelle de volatilité qui va de 0 à 5, la valeur 0 correspondant à une poudre non volatile et 5 à une poudre très volatile.

Les résultats de ces différents essais sont donnés dans les trois dernières lignes du tableau I ci-après.

### EXEMPLES 1 et 2 :

**TABLEAU I**

| Matières premières | Exemple 1 | Exemple 2 |
|---|---|---|
| Persulfate de potassium | 45 | 45 |
| Persulfate de sodium | 7,5 | 7,5 |
| Métasilicate de sodium anhydre | 11 | 11 |
| Chlorure d'ammonium | 5 | 5 |
| Acide éthylènediaminetétraacétique | 1 | 1 |
| Gomme de Guar | 1 | 1 |
| Silice hydrophile | 3 | 3 |
| Mélange de stéarate de glycérol/stéarate de potassium (93/7 en poids) | 0,5 | 0,5 |
| Carboxyméthyl cellulose de sodium | 1,7 | 1,7 |
| Oxyde de titane | 0,5 | 0,5 |
| Silice colloïdale | 13,8 | 13,8 |
| PPG (poids moléculaire = 2 000) | 10 | - |
| PPG (poids moléculaire = 6 000) | - | 10 |
| Granulométrie moyenne (en µm) | 200 | 200 |
| Temps de dissolution (mn) | 6 | 5 |
| Volatilité | 0,5 | 0,5 |

### EXEMPLES 3 et 4 (comparatifs)

On a effectué des essais comparatifs avec des poudres granulées par le procédé décrit pour les exemples 1 et 2, en utilisant comme liant pulvérisé une polyvinylpyrrolidone (PVP) et un polyéthylèneglycol (PEG) respectivement pour les exemples 3 et 4. On a effectué sur les granules obtenus les mêmes essais que dans les exemples 1 et 2. La formulation de la composition granulée est donnée en % en poids et les résultats des essais sont donnés dans le tableau II ci-après :

**TABLEAU II**

| Matières premières | Exemple 3 | Exemple 4 |
|---|---|---|
| Persulfate de potassium | 40 | 50 |
| Persulfate de sodium | 9 | 7,5 |
| Métasilicate de sodium anhydre | 10 | 10,8 |
| Chlorure d'ammonium | 5 | 4 |
| Acide éthylènediaminetétraacétique | 2 | 1 |
| Gomme de Guar | - | 1 |
| Silice hydrophile | - | 3 |
| PVP (poids moléculaire = 40 000) | 12 | - |
| PEG (poids moléculaire = 400) | - | 12 |
| Carboxyméthylcellulose de sodium | 5 | - |
| Mélange de stéarate de glycérol/stéarate de potassium (93/7 en poids) | - | 0,5 |
| Silice colloïdale | 17 | 10,2 |
| Granulométrie (en µm) | > 1 000 | 65 à 1 000 |
| Temps de dissolution (mn) | > 60 | 30 |
| Volatilité | 1 | 1,5 |

Ces essais montrent que, lorsqu'on utilise un autre liant que le PPG, les temps de dissolution des granules sont plus élevés, la granulomérie des granules est trop élevée ou trop dispersée et la formation de poussière est relativement élevée.

## Revendications

1. Composition granulée, à base de dérivés peroxydés, utilisable notamment pour la décoloration des cheveux, dans laquelle les granules ont été obtenus par granulation d'un mélange de ses différents constituants au moyen d'un liant, caractérisée par le fait que le liant comprend du polypropylèneglycol ayant un poids moléculaire compris entre 200 et 10 000, les granules ayant une granulométrie comprise entre 65 µm et 800 µm.

2. Composition selon la revendication 1, caractérisée par le fait que le polypropylèneglycol a un poids moléculaire compris entre 2 000 et 8 000.

3. Composition selon l'une des revendications 1 ou 2, caractérisée par le fait que le polypropylèneglycol est mélangé à au moins un polyalkylèneglycol ayant un groupe alkylène en C₂ ou C₄.

4. Composition selon la revendication 3, caractérisée par le fait que le polyalkylèneglycol en C₂ est un polyéthylèneglycol ayant un poids moléculaire compris entre 200 et 4 000.

5. Composition selon la revendication 3, caractérisée par le fait que le polyalkylèneglycol en C₄ est un polybutylèneglycol ayant un poids moléculaire compris entre 200 et 30 000.

6. Composition selon l'une des revendications 1 à 5, caractérisée par le fait que la concentration globale en liant ne dépasse pas 25 % en poids par rapport au poids total de la composition.

7. Composition selon la revendication 6, caractérisée par le fait que la concentration en polypropylèneglycol est comprise entre 10 et 20 % en poids par rapport au poids total de la composition.

8. Composition selon l'une des revendications 3 à 5, caractérisée par le fait que la concentration en polyalkylèneglycol(s) ayant un groupe alkylène en C₂ ou C₄ ne dépasse pas 5 % en poids par rapport au poids total de la composition.

9. Composition selon l'une des revendications 1 à 8, caractérisée par le fait qu'elle contient au moins un agent lubrifiant pris dans le groupe formé par les dérivés de cellulose et les stéarates.

10. Composition selon la revendication 9, caractérisée par le fait que l'agent lubrifiant comporte au moins un dérivé de cellulose choisi dans le groupe formé par la carboxyméthylcellulose de sodium et l'hydroxypropylméthylcellulose.

11. Composition selon l'une des revendications 9 ou 10, caractérisée par le fait que la quantité de dérivé(s) de cellulose présent(s) comme agent lubrifiant représente 1 à 10 % en poids du poids total de la composition.

12. Composition selon la revendication 9, caractérisée par le fait que l'agent lubrifiant comporte au moins un stéarate choisi dans le groupe formé par les stéarates alcalins, alcalino-terreux et les stéarates de polyol.

13. Composition selon l'une des revendications 9 ou 12, caractérisée par le fait que la quantité de stéarate(s) présent(s) comme agent lubrifiant est comprise entre 0,1 et 5 % en poids par rapport au poids total de la composition.

14. Procédé de préparation de la composition selon l'une des revendications 1 à 13, caractérisé par le fait qu'on prépare un lit mobile des différents constituants pulvérulents du mélange à granuler, on pulvérise sur le lit mobile une solution de polypropylèneglycol dans un solvant, le polypropylèneglycol étant éventuellement mélangé à au moins un polyalkylèneglycol en C₂ ou C₄, et on élimine le solvant.

15. Procédé selon la revendication 14, caractérisé par le fait qu'on choisit le solvant dans le groupe formé par les alcanols en C₁-C₄, le chlorure de méthylène, le chlorure d'éthylène et leurs mélanges.

16. Procédé selon l'une des revendications 14 ou 15, caractérisé par le fait que le mélange de constituants pulvérulents est homogénéisé avant granulation.

17. Procédé selon l'une des revendications 14 à 16, destiné à préparer une composition selon la revendication 9, caractérisé par le fait que le mélange à granuler comprend un agent lubrifiant.

18. Procédé selon l'une des revendications 14 à 17, caractérisé par le fait que l'on obtient le lit mobile du mélange à granuler dans une cuve à l'aide d'agitateur(s) ou dans un tambour rotatif.

19. Procédé selon l'une des revendications 14 à 17, caractérisé par le fait que l'on obtient le lit mobile du mélange à granuler par fluidisation dans un courant gazeux ascendant.

20. Procédé selon l'une des revendications 14 à 19, caractérisé par le fait qu'on élimine le solvant thermiquement et/ou sous pression réduite.

## Claims

1. Granulated composition based on peroxidized derivatives, which may especially be used for bleaching hair, in which the granules have been obtained by granulation of a mixture of its various constituents by means of a binder, characterized in that the binder comprises polypropylene glycol of molecular weight between 200 and 10,000, the granules having a particle size between 65 µm and 800 µm.

2. Composition according to Claim 1, characterized in that the polypropylene glycol has a molecular weight between 200 and 8000.

3. Composition according to either of Claims 1 and 2, characterized in that the polypropylene glycol is mixed with at least one polyalkylene glycol having a C₂ or C₄ alkylene group.

4. Composition according to Claim 3, characterized in that the C₂ polyalkylene glycol is a polyethylene glycol of molecular weight between 200 and 4,000.

5. Composition according to Claim 3, characterized in that the C₄ polyalkylene glycol is a polybutylene glycol of molecular weight between 200 and 30,000.

6. Composition according to one of Claims 1 to 5, characterized in that the overall binder concentration does not exceed 25% by weight relative to the total weight of the composition.

7. Composition according to Claim 6, characterized in that the concentration of polypropylene glycol is between 10 and 20% by weight relative to the total weight of the composition.

8. Composition according to one of Claims 3 to 5, characterized in that the concentration of polyalkylene glycol(s) having a C₂ or C₄ alkylene group does not exceed 5% by weight relative to the total weight of the composition.

9. Composition according to one of Claims 1 to 8, characterized in that it contains at least one lubricating agent taken from the group formed by cellulose derivatives and stearates.

10. Composition according to Claim 9, characterized in that the lubricating agent contains at least one cellulose derivative chosen from the group formed by sodium carboxymethyl cellulose and hydroxypropylmethyl cellulose.

11. Composition according to either of Claims 9 or 10, characterized in that the quantity of cellulose derivative(s) present as lubricating agent represents from 1 to 10% by weight of the total weight of the composition.

12. Composition according to Claim 9, characterized in that the lubricating agent includes at least one stearate chosen from the group formed by alkali metal stearates, alkaline-earth metal stearates and polyol stearates.

13. Composition according to either of Claims 9 or 12, characterized in that the quantity of stearate(s) present as lubricating agent is between 0.1 and 5% by weight relative to the total weight of the composition.

14. Process for the preparation of the composition according to one of Claims 1 to 13, characterized in that a moving bed of the various pulverulent constituents of the mixture to be granulated is prepared and polypropylene glycol, which is optionally mixed with at least one C₂ or C₄ polyalkylene glycol, is sprayed in the form of a solution in a solvent onto the moving bed, and the solvent is removed.

15. Process according to Claim 14, characterized in that the solvent for the second binder is chosen from the group formed by C₁-C₄ alkanols, methylene chloride, ethylene chloride and their mixtures.

16. Process according to either of Claims 14 and 15, characterized in that the mixture of pulverulent constituents is homogenized before granulation.

17. Process according to one of Claims 1 to 16, intended for the preparation of a composition according to Claim 9, characterized in that the mixture to be granulated contains a lubricating agent.

18. Process according to one of Claims 14 to 17, characterized in that the moving bed for the mixture to be granulated is obtained in a tank with the aid of (a) stirrer(s) or in a rotating drum.

19. Process according to one of Claims 14 to 17, characterized in that the moving bed for the mixture to be granulated is obtained by fluidization in a stream of ascending gas.

20. Process according to one of Claims 14 to 19, characterized in that the solvent is removed thermally and/or under reduced pressure.

## Patentansprüche

1. Granuliertes Mittel auf Basis von Peroxydderivaten, welches insbesondere zur Haarentfärbung brauchbar ist, worin die Granula durch Granulierung eines Gemisches seiner verschiedenen Bestandteile mit Hilfe eines Bindemittels erhalten wurden,
**dadurch gekennzeichnet**, daß das Bindemittel ein Polypropylenglykol mit einem Molekulargewicht von 200 - 10000 umfaßt, wobei die Granula eine Korngröße von 65 µm bis 800 µm aufweisen.

2. Mittel nach Anspruch 1,
**dadurch gekennzeichnet**, daß das Polypropylenglykol ein Molekulargewicht von 2000 bis 8000 aufweist.

3. Mittel nach einem der Ansprüche 1 oder 2,
**dadurch gekennzeichnet**, daß das Polypropylenglykol ein Gemisch aus wenigstens einem Polyalkylenglykol mit einer C₂- oder C₄-Alkylengruppe ist.

4. Mittel nach Anspruch 3,
**dadurch gekennzeichnet**, daß das C₂-Polyalkylenglykol ein Polyethylenglykol mit einem Molekulargewicht von 200 bis 4000 ist.

5. Mittel nach Anspruch 3,
**dadurch gekennzeichnet**, daß das C₄-Polyalkylenglykol ein Polybutylenglykol mit einem Molekulargewicht von 200 bis 30000 ist.

6. Mittel nach einem der Ansprüche 1 - 5,
**dadurch gekennzeichnet**, daß die Gesamtkonzentration an Bindemittel 25 Gew.-%, bezogen auf das Gesamtgewicht des Mittels, nicht übersteigt.

7. Mittel nach Anspruch 6,
**dadurch gekennzeichnet**, daß die Konzentration an Polypropylenglykol 10 - 20 Gew.-%, bezogen auf das Gesamtgewicht des Mittels, umfaßt.

8. Mittel nach einem der Ansprüche 3 bis 5,
**dadurch gekennzeichnet**, daß die Konzentration an Polyalkylenglykol(en) mit einer C₂- oder C₄-Alkylengruppe 5 Gew.-%, bezogen auf das Gesamtgewicht des Mittels, nicht übersteigt.

9. Mittel nach einem der Ansprüche 1 bis 8,
**dadurch gekennzeichnet**, daß es wenigstens ein Gleitmittel, ausgewählt unter Cellulosederivaten und Stearaten, enthält.

10. Mittel nach Anspruch 9,
**dadurch gekennzeichnet**, daß das Gleitmittel wenigstens ein Cellulosederivat, ausgewählt unter Natriumcarboxymethylcellulose und Hydroxypropylmethylcellulose, umfaßt.

11. Mittel nach einem der Ansprüche 9 oder 10,
**dadurch gekennzeichnet**, daß die Menge an Cellulosederivat(en), das (die) als Gleitmittel zugegen ist (sind), 1 bis 10 Gew.-% des Gesamtgewichts des Mittels ausmacht.

12. Mittel nach Anspruch 9,
**dadurch gekennzeichnet**, daß das Gleitmittel wenigstens ein Stearat, ausgewählt unter Alkali-Erdalkali- und Polyolstearaten, umfaßt.

13. Mittel nach einem der Ansprüche 9 oder 12,
**dadurch gekennzeichnet**, daß die Menge an Stearat(en), das (die) als Gleitmittel zugegen ist (sind), 0,1 bis 5 Gew.-%, bezogen auf das Gesamtgewicht des Mittels, ausmacht.

14. Verfahren zur Herstellung des Mittels nach einem der Ansprüche 1 - 13,
**dadurch gekennzeichnet**, daß man eine mobile Schicht aus den verschiedenen pulverförmigen Bestandteilen des zu granulierenden Gemisches herstellt, auf der mobilen Schicht eine Lösung von Polypropylenglykol in einem Lösungsmittel zerstäubt, wobei das Polypropylenglykol gegebenenfalls mit wenigstens einem C₂- oder C₄-Polyalkylenglykol vermischt wird, und man das Lösungsmittel entfernt.

15. Verfahren nach Anspruch 14,
**dadurch gekennzeichnet**, daß man das Lösungsmittel unter C₁-C₄-Alkanolen, Methylenchlorid, Ethylenchlorid und deren Gemischen auswählt.

16. Verfahren nach einem der Ansprüche 14 oder 15,
**dadurch gekennzeichnet**, daß das Gemisch aus pulverförmigen Bestandteilen vor Granulierung homogenisiert wird.

17. Verfahren nach einem der Ansprüche 14 bis 16, das dazu dient, ein Mittel nach Anspruch 9 herzustellen,
**dadurch gekennzeichnet**, daß das zu granulierende Gemisch ein Gleitmittel umfaßt.

18. Verfahren nach einem der Ansprüche 14 bis 17,
**dadurch gekennzeichnet**, daß man die mobile Schicht des zu granulierenden Gemisches in einem Behälter mit Hilfe eines oder mehrerer Rührer oder in einem Rührkessel erhält.

19. Verfahren nach einem der Ansprüche 14 bis 17,
**dadurch gekennzeichnet**, daß man die mobile Schicht der zu granulierenden Mischung durch Fluidisation in einem aufsteigenden Gasstrom erhält.

20. Verfahren nach einem der Ansprüche 14 bis 19,
**dadurch gekennzeichnet**, daß man das Lösungsmittel thermisch und/oder unter vermindertem Druck entfernt.
